# FASCICULE DE BREVET EUROPEEN

(11) **EP 1 855 005 B1**
(45) Date de publication et mention de la délivrance du brevet: **28.01.2009**
(21) Numéro de dépôt: 07290571.4
(22) Date de dépôt: 07.05.2007
(51) Int. Cl.: F04C 2/10, F01C 21/02, F04C 2/08, F04C 15/00

(54) **Pompe volumétrique rotative à encombrement radial réduit**
Rotationsverdrängerpumpe mit beschränktem radialem Raumbedarf
Rotary positive displacement pump with reduced radial space requirement

(30) Priorité: 12.05.2006 FR 0604206
(43) Date de publication de la demande: 14.11.2007
(73) Titulaire: Groupement Coeur Artificiel Total Carpentier Matra Carmat, 78140 Velizy Villacoublay (FR)
(72) Inventeur: Grimmé, Marc, 75019 Paris (FR); Parquet, Jean-Marc, 95330 Domont (FR); Carpentier, Alain, 75116 Paris (FR); Wartelle, Claude, 60270 Gouvieux (FR)
(74) Mandataire: Bonnetat, Christian

(56) Documents cités:
- EP-A1- 0 324 669
- EP-A2- 0 560 709
- DE-A1- 3 709 901
- DE-U1- 8 121 531
- FR-A1- 2 760 973

## Description

La présente invention concerne une pompe volumétrique rotative à encombrement radial réduit.

Par les documents US-2 458 958, FR-1 504 705, EP-0 560 709, FR 2 760 973 et DE-81 21 531 U1, on connaît déjà une pompe volumétrique rotative comportant :
- un carter dans lequel sont agencées une chambre de pompage, ainsi qu'une entrée et une sortie radiales de fluide pour ladite chambre de pompage ;
- un engrenage extérieur à denture interne, disposé dans ladite chambre de pompage et présentant la forme d'une cage d'écureuil pourvue de deux flasques d'extrémité reliés par des barreaux longitudinaux formant ladite denture interne, ledit engrenage extérieur étant au moins en partie démontable pour pouvoir y introduire un engrenage intérieur;
- des moyens de rotation permettant la rotation dudit engrenage extérieur à l'intérieur de ladite chambre de pompage, autour d'un premier axe et comportant au moins un palier agencé centralement dans un flasque d'extrémité dudit engrenage extérieur ;
- ledit engrenage intérieur, logé à l'intérieur dudit engrenage extérieur et pourvu d'une denture externe engrenant avec ladite denture interne de l'engrenage extérieur, ledit engrenage intérieur tournant autour d'un second axe, parallèle et écarté dudit premier axe, et ladite denture externe comportant un nombre de dents inférieur d'une unité au nombre de dents de la denture interne dudit engrenage extérieur ;
- un moteur d'entraînement en rotation dudit engrenage intérieur autour dudit second axe; et
- ladite chambre de pompage comporte une paroi amovible, apte à être fixée sur ledit carter pour obturer ladite chambre de pompage, et un premier palier desdits moyens de rotation est agencé dans ladite paroi amovible.

La présente invention a pour objet de perfectionner une pompe volumétrique rotative de ce type, afin d'en réduire l'encombrement radial, tout en permettant le montage et le démontage dudit engrenage intérieur dans ledit engrenage extérieur.

A cette fin, selon l'invention, la pompe volumétrique rotative du type décrit ci-dessus est remarquable en ce que :
- un second palier desdits moyens de rotation est agencé dans une paroi fixe de ladite chambre de pompage ;
- ledit carter comporte un logement pour ledit moteur et ladite paroi fixe, faisant partie intégrale du carter, sépare ledit logement de ladite chambre de pompage ;
- l'arbre du moteur entraînant l'engrenage intérieur en rotation traverse ladite paroi fixe à l'intérieur dudit second palier ; et
- ledit arbre du moteur est creux et traversé avec jeu radial par un tirant servant à fixer ladite paroi amovible audit carter.

De préférence, au moins l'un des flasques d'extrémité dudit engrenage extérieur est démontable.

Par ailleurs, il est avantageux d'agencer, sur ledit tirant, des paliers pour ledit arbre du moteur, c'est-à-dire pour ledit engrenage intérieur.

Dans le cas où la pompe conforme à la présente invention est destinée à être immergée dans un liquide hydraulique, ledit tirant peut lui-même être creux pour laisser y pénétrer ledit liquide et la paroi latérale dudit tirant peut être percée d'ouvertures. Ainsi, il est possible de lubrifier lesdits paliers de l'engrenage intérieur, portés par ledit tirant, par ledit liquide hydraulique.

Pour éviter les risques de cavitation et les pertes turbulentes dans le fluide mû par la pompe, il est avantageux que ladite entrée de fluide radiale et/ou ladite sortie de fluide radiale s'étende(nt) d'un flasque à l'autre dudit engrenage extérieur.

Aux mêmes fins, la denture interne de l'engrenage extérieur et la denture interne de l'engrenage extérieur comportent, au plus, cinq et quatre dents respectivement. Ainsi, le diamètre extérieur de ladite pompe peut être réduit, ce qui réduit en conséquence les vitesses du fluide en périphérie de ladite pompe.

Grâce aux particularités décrites ci-dessus, on comprendra aisément que la pompe conforme à la présente invention peut être miniaturisée tout en ayant un débit réversible.

Selon un exemple d'application, la pompe miniaturisée conforme à la présente invention peut être incorporée dans une prothèse cardiaque implantable dans la cavité péricardique d'un patient.

Ainsi, une telle prothèse cardiaque, qui est apte à remplacer les ventricules gauche et droit naturels dudit patient après ablation de ceux-ci et qui comporte un corps dans lequel sont agencés des ventricules gauche et droit artificiels actionnés par au moins un actionneur hydraulique, est remarquable en ce que ledit actionneur hydraulique est une pompe volumétrique telle que celle spécifiée ci-dessus.

Si, comme cela est usuel, les ventricules artificiels comportent un couvercle d'obturation rapporté audit corps de prothèse, il est avantageux, selon l'invention, qu'une telle pompe volumétrique soit reliée à un tel couvercle d'obturation.

Les figures du dessin annexé feront bien comprendre comment l'invention peut être réalisée. Sur ces figures, des références identiques désignent des éléments semblables.
La figure 1 est une vue de dessus d'un exemple de réalisation à faible encombrement de la pompe volumétrique rotative conforme à la présente invention.
La figure 2 est une coupe transversale selon la ligne II-II de la figure 1.
La figure 3 est une coupe axiale selon la ligne III-III de la figure 2.
La figure 4 est une vue en perspective de la pompe des figures 1 à 3, de laquelle ont été éliminés les engrenages extérieur et intérieur.
La figure 5 est une vue en perspective de l'engrenage extérieur.
La figure 6 est une vue en perspective de l'ensemble de l'engrenage extérieur et de l'engrenage intérieur.
La figure 7 est une vue en perspective, schématique et partielle, d'une prothèse cardiaque équipée d'un actionneur hydraulique.
La figure 8 est une vue de dessus de la pompe des figures 1 à 3, montée sur le couvercle d'un ventricule.
La figure 9 est une coupe transversale selon la ligne IX-IX de la figure 8.

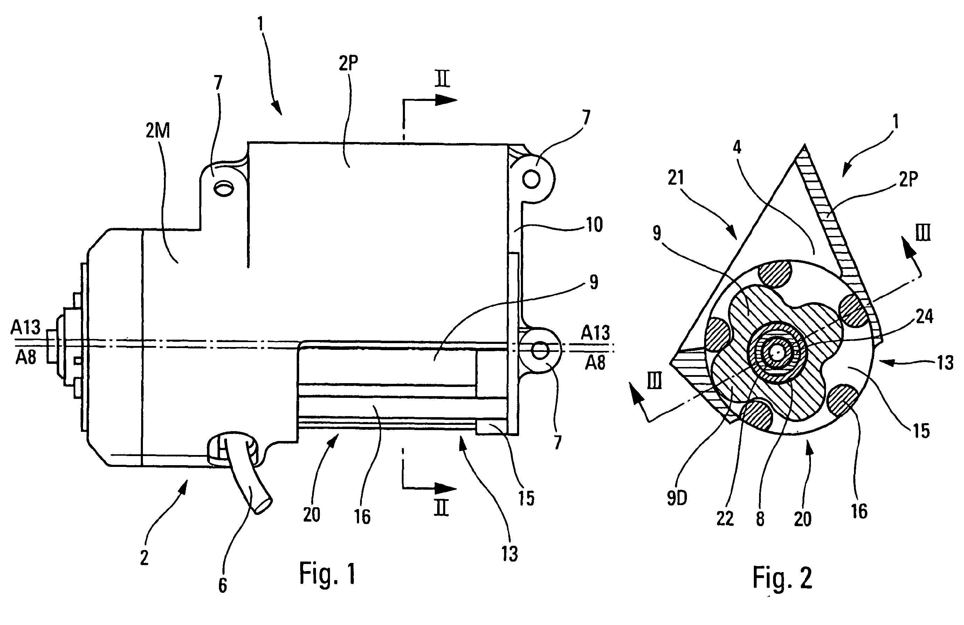

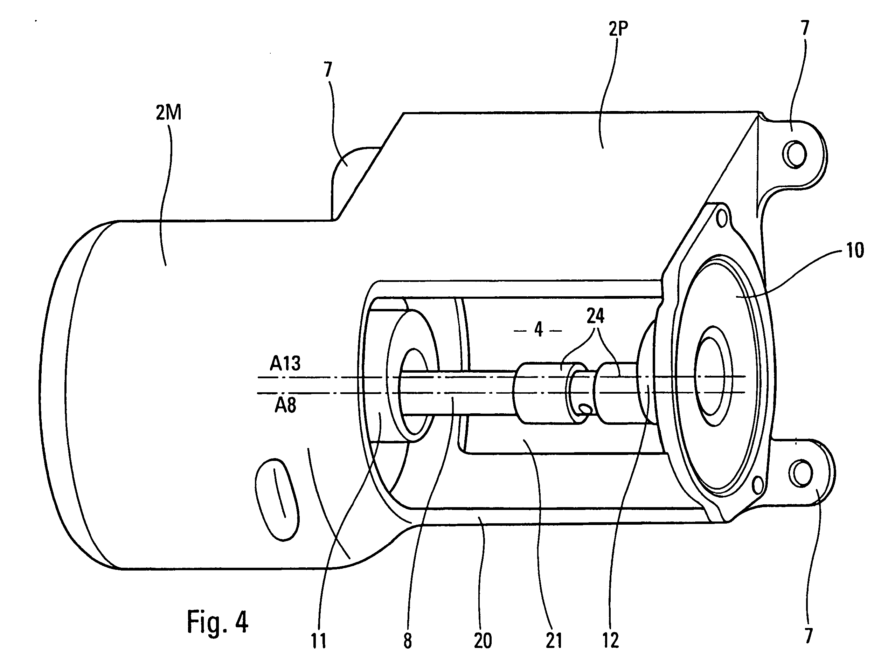

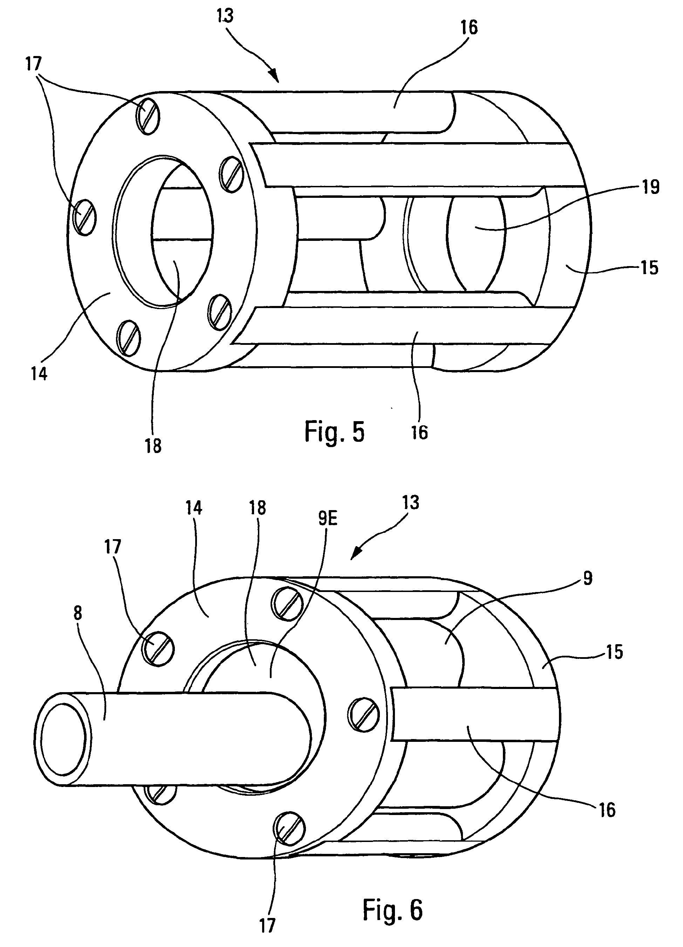

La pompe volumétrique rotative 1, conforme à la présente invention et illustrée par les figures 1 à 6, comporte un carter 2 comprenant une partie 2M, dans laquelle est logé un moteur électrique 3, et une partie 2P, dans laquelle est agencée une chambre de pompage 4. Les parties 2M et 2P du carter 2 sont séparées l'une de l'autre par une paroi interne fixe 5 faisant partie intégrante dudit carter 2. Le moteur électrique 3 est alimenté par un câble électrique 6 traversant la partie de carter 2M et ledit carter 2 est pourvu de pattes de fixation 7.

L'arbre rotatif 8 du moteur électrique 3 présente un axe A8 et il traverse la paroi interne fixe 5 pour pénétrer dans la chambre de pompage 4. Sur la partie dudit arbre 8, logée dans la chambre de pompage 4, est calé un engrenage 9 allongé, à quatre dents arrondies externes 9D.

La partie 2P du carter 2, formant ladite chambre de pompage 4, est ouverte à son extrémité opposée à la partie 2M et peut être obturée de façon étanche par une paroi amovible 10, apte à être fixée audit carter 2.

Dans ladite paroi interne fixe 5 et dans ladite paroi amovible 10 sont respectivement agencés, du côté de ladite chambre de pompage 4 et en regard l'un de l'autre, des paliers 11 et 12 définissant un axe A13 pour un engrenage 13 logé dans ladite chambre de pompage 4.

L'axe A13 est parallèle à l'axe A8, mais décalé par rapport à celui-ci.

L'engrenage 13 présente la forme d'une cage d'écureuil pourvue de deux flasques d'extrémité 14 et 15, reliés par cinq barreaux longitudinaux 16 formant des dents arrondies internes pour ledit engrenage 13. Dans l'exemple représenté sur les figures, les barreaux 16 sont solidaires du flasque 15, alors que le flasque 14 est amovible, grâce à un montage par vis 17, lui permettant d'être séparé du flasque d'extrémité 15 et des barreaux 16.

Les flasques 14 et 15 sont respectivement pourvus d'évidements circulaires centraux 18 et 19, aptes à coopérer respectivement avec les paliers 11 et 12 pour permettre la rotation dudit engrenage 13 à l'intérieur de ladite chambre de pompage 4.

L'engrenage 9 est logé à l'intérieur de l'engrenage 13, avec ses faces d'extrémité 9E respectivement au voisinage des flasques 14 et 15 et l'arbre 8 traversant l'évidement circulaire central 18 du flasque 14. On comprendra aisément que, grâce à l'amovibilité du flasque 14, il est aisé de monter l'engrenage 9 à l'intérieur de l'engrenage 13.

Lorsque l'engrenage 13 est monté sur les paliers 11 et 12 et que l'engrenage 9 est disposé à l'intérieur dudit engrenage 13 et calé sur l'arbre 8, les dents arrondies 9D et 16 respectives desdits engrenages sont aptes à coopérer, pour produire un pompage.

Par ailleurs, la partie 2P du carter 2 définissant la chambre de pompage 4 comporte deux ouvertures 20 et 21 en regard, s'étendant sur la longueur de l'engrenage 9 et aptes à servir respectivement d'entrée et de sortie radiales pour le fluide à pomper.

Ainsi, lorsque le moteur 3, par l'intermédiaire de l'arbre 8, entraîne la rotation de l'engrenage 9 autour de l'axe A8, les dents externes arrondies 9D dudit engrenage 9 coopèrent avec les dents internes arrondies 16 de l'engrenage 13, pour entraîner ce dernier en rotation autour de l'axe A13 des paliers 11 et 12. Ce faisant, du fluide est aspiré à travers l'entrée 20 et refoulé à travers la sortie 21.

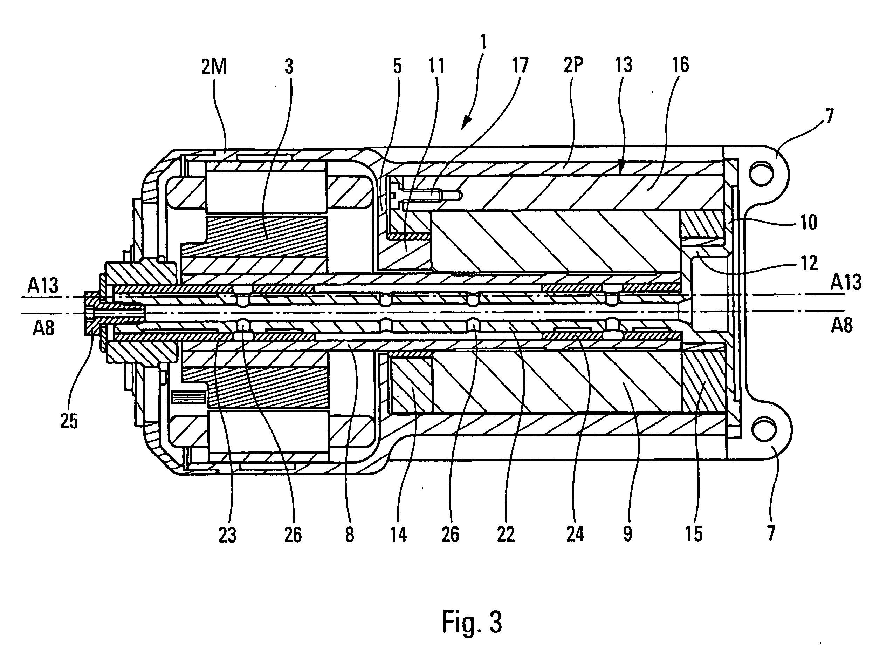

Comme on peut le voir clairement sur la figure 3, l'arbre 8 du moteur 3 et de l'engrenage intérieur 9 est creux et il est traversé avec jeu par un tirant 22, solidaire de la paroi amovible 10. Des paliers 23, 24 sont portés par le tirant 22 et servent à la rotation de l'engrenage intérieur 9. Une vis 25, engagée à l'extrémité libre dudit tirant 22, prend appui sur l'extrémité du carter 2, opposée à la paroi amovible 10, pour plaquer ladite paroi amovible 10 contre ledit carter.

La vis 25 et le tirant 22 sont creux et des orifices 26 sont percés dans la paroi latérale du tirant 22.

Ainsi, lorsque la pompe 1 est immergée dans un liquide hydraulique, celui-ci peut lubrifier lesdits paliers 23, 24 de l'engrenage intérieur 9.

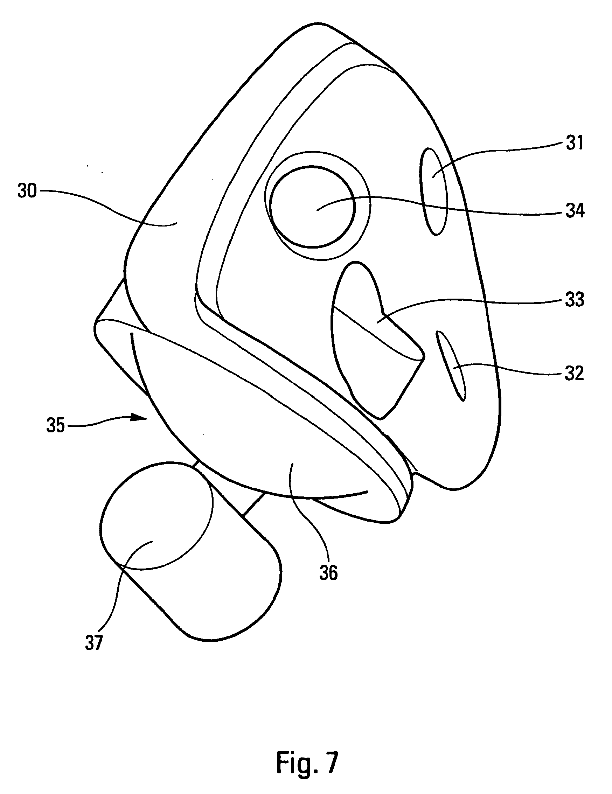

Sur la figure 7, on a représenté schématiquement en perspective le corps 30 d'une prothèse cardiaque implantable après ablation des ventricules naturels d'un patient et pourvue d'orifices de raccord 31 à 34, respectivement à l'oreillette droite, à l'oreillette gauche, à l'aorte et à l'artère pulmonaire dudit patient. Sur cette figure 7, on a indiqué, de l'extérieur dudit corps 30, l'emplacement d'un ventricule artificiel 35, obturé par un couvercle 36 portant un actionneur 37 pour ledit ventricule.

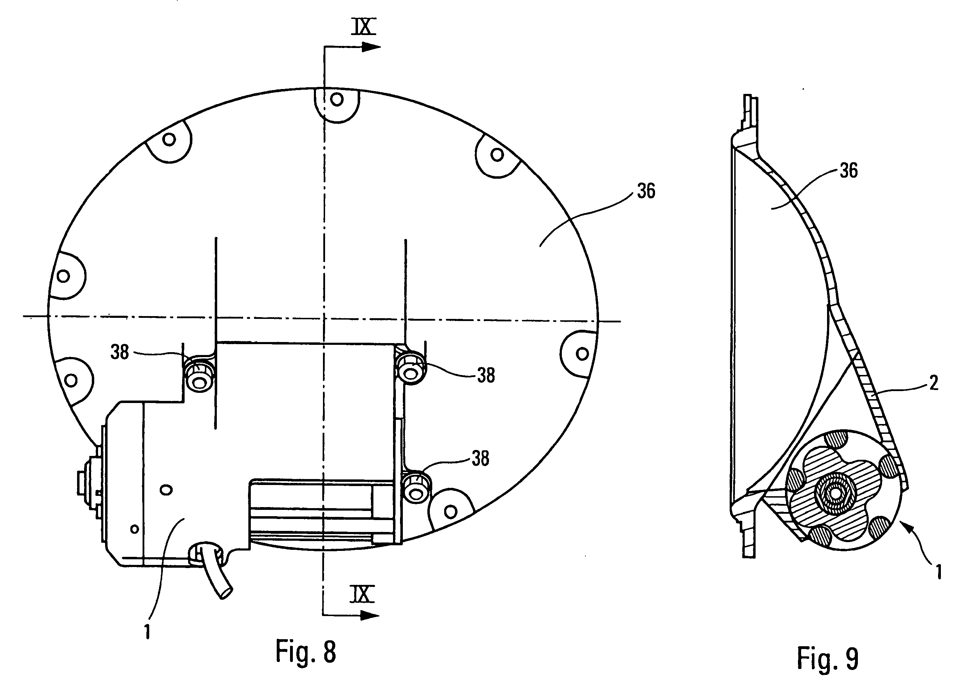

Les figures 8 et 9 montrent un mode de réalisation du couvercle 36 du ventricule artificiel 35, sur lequel ledit actionneur 37 est formé par la pompe 1 conforme à la présente invention, ladite pompe 1 étant fixée audit couvercle 36 par des boulons 38 traversant les pattes 7.

## Revendications

1. Pompe volumétrique rotative, comportant :
- un carter (2) dans lequel sont agencées une chambre de pompage (4), ainsi qu'une entrée (20) et une sortie (21) radiales de fluide pour ladite chambre de pompage (4) ;
- un engrenage extérieur (13) à denture interne (16), disposé dans ladite chambre de pompage (4) et présentant la forme d'une cage d'écureuil pourvue de deux flasques d'extrémité (14, 15) reliés par des barreaux longitudinaux formant ladite denture interne, ledit engrenage extérieur (13) étant au moins en partie démontable pour pouvoir y introduire un engrenage intérieur;
- des moyens de rotation (11, 12) permettant la rotation dudit engrenage extérieur (13) à l'intérieur de ladite chambre de pompage (4), autour d'un premier axe (A 13) et comportant au moins un palier (11, 12) agencé centralement dans un flasque d'extrémité (14, 15) dudit engrenage extérieur (13) ;
- ledit engrenage intérieur (9), logé à l'intérieur dudit engrenage extérieur (13) et pourvu d'une denture externe (9D) engrenant avec ladite denture interne (16) de l'engrenage extérieur (13), ledit engrenage intérieur (9) tournant autour d'un second axe (A8), parallèle et écarté dudit premier axe (A13), et ladite denture externe (9D) comportant un nombre de dents inférieur d'une unité au nombre de dents de la denture interne dudit engrenage extérieur (13);
- un moteur (3) d'entraînement en rotation dudit engrenage intérieur (9) autour dudit second axe (A8); et
- ladite chambre de pompage (4) comporte une paroi amovible (10), apte à être fixée sur ledit carter (2) pour obturer ladite chambre de pompage (4), et un premier palier (12) desdits moyens de rotation est agencé dans ladite paroi amovible (10) ;
**caractérisée en ce que** :
- un second palier (11) desdits moyens de rotation est agencé dans une paroi fixe (5)*,* faisant partie intégrale du carter, de ladite chambre de pompage (4) ;
- ledit carter (2) comporte un logement (2M) pour ledit moteur (3) et ladite paroi fixe (5) sépare ledit logement (2M) de ladite chambre de pompage (4) ;
- l'arbre (8) du moteur (3) entraînant l'engrenage intérieur (9) en rotation traverse ladite paroi fixe (5) à l'intérieur dudit second palier (11) ; et
- ledit arbre (8) du moteur (3) est creux et traversé avec jeu radial par un tirant (22) servant à fixer ladite paroi amovible (10) audit carter (2).

2. Pompe volumétrique selon la revendication 1,
**caractérisée en ce qu'**au moins l'un des flasques d'extrémité (14, 15) dudit engrenage extérieur (13) est démontable.

3. Pompe volumétrique selon l'une des revendications 1 ou 2,
**caractérisée en ce que** des paliers (23, 24) pour ledit engrenage intérieur (9) sont agencés sur ledit tirant (22).

4. Pompe volumétrique selon la revendication 3, destinée à être immergée dans un liquide hydraulique,
**caractérisée en ce que** ledit tirant (22) est creux pour laisser y pénétrer ledit liquide et **en ce que** la paroi latérale dudit tirant (22) est percée d'ouvertures (26).

5. Pompe volumétrique selon l'une quelconque des revendications 1 à 4,
**caractérisée en ce que** ladite entrée de fluide radiale (20) s'étend d'un flasque à l'autre dudit engrenage extérieur (13).

6. Pompe volumétrique selon l'une quelconque des revendications 1 à 5,
**caractérisée en ce que** ladite sortie de fluide radiale (21) s'étend d'un flasque à l'autre dudit engrenage extérieur (13).

7. Pompe volumétrique selon l'une quelconque des revendications 1 à 6,
**caractérisée en ce que** la denture interne (16) de l'engrenage extérieur (13) comporte, au plus cinq dents, alors que la denture externe de l'engrenage intérieur (9) comporte au plus quatre dents (9D).

8. Prothèse cardiaque implantable dans la cavité péricardique d'un patient, ladite prothèse étant apte à remplacer les ventricules gauche et droit naturels dudit patient après ablation de ceux-ci et comportant un corps (30) dans lequel sont agencés des ventricules gauche et droit artificiels (35) actionnés par au moins un actionneur hydraulique (37),
**caractérisée en ce que** ledit actionneur hydraulique est une pompe volumétrique (1) telle que celle spécifiée sous l'une quelconque des revendications 1 à 7.

9. Prothèse cardiaque selon la revendication 8, dans laquelle les ventricules artificiels (35) comportent un couvercle d'obturation (36) rapporté audit corps (30),
**caractérisée en ce que** la pompe volumétrique est reliée à un tel couvercle d'obturation (36).

## Claims

1. A rotary displacement pump comprising:
- a casing (2) in which there are a pumping chamber (4) and a radial fluid inlet (20) and outlet (21) for said pumping chamber (4);
- an outer gear (13) with an internal toothset (16) positioned inside said pumping chamber (4) and displaying the shape of a squirrel cage provided with two end plates (14, 15) which are connected by longitudinal bars that form said internal toothset, said outer gear (13) being at least partially dismantleable, in order to be able to introduce an inner gear therein ;
- rotation means (11, 12) allowing said outer gear (13) to rotate inside said pumping chamber (4) about a first axis (A13) and comprising at least one bearing (11, 12) positioned centrally in an end plate (14, 15) of said outer gear (13);
- said inner gear (9), housed inside said outer gear (13) and provided with an external toothset (9D) meshing with said internal toothset (16) of the outer gear (13), said inner gear (9) rotating about a second axis (A8), parallel to and distant from said first axis (A13), and said external toothset (9D) comprising a number of teeth lower by one unit than the number of teeth of the internal toothset of said outer gear (13);
- a motor (3) for driving the rotation of said inner gear (9) about said second axis (A8); and
- said pumping chamber (4) comprises a removable wall (10) capable of being fixed to said casing (2) to close off said pumping chamber (4), and a first bearing (12) of said rotation means is positioned in said removable wall (10);
**characterized in that** :
- a second bearing (11) of said rotation means is positioned in a fixed wall (5) of said pumping chamber (4), integral with the casing;
- said casing (2) comprises a housing (2M) for said motor (3) and said fixed wall (5) separates said housing (2M) from said pumping chamber (4);
- the shaft (8) of the motor (3) driving the rotation of the inner gear (9) passes through said fixed wall (5) inside said second bearing (11); and
- said shaft (8) of the motor (3) is hollow and through it there passes, with radial clearance, a tie rod (22) used to fix said removable wall (10) to said casing (2).

2. The displacement pump as claimed in claim 1,
**characterized in that** at least one of the end plates (14, 15) of said outer gear (13) is dismantleable.

3. The displacement pump as claimed in one of claims 1 and 2,
**characterized in that** bearings (23, 24) for said inner gear (9) are positioned on said tie rod (22).

4. The displacement pump as claimed in claim 3, intended to be immersed in a hydraulic liquid,
**characterized in that** said tie rod (22) is hollow so that said liquid can enter it, and **in that** the side wall of said tie rod (22) is pierced with orifices (26).

5. The displacement pump as claimed in any one of claims 1 to 4,
**characterized in that** said radial fluid inlet (20) extends from one end plate of said outer gear (13) to the other.

6. The displacement pump as claimed in any one of claims 1 to 5,
**characterized in that** said radial fluid outlet (21) extends from one end plate of said outer gear (13) to the other.

7. The displacement pump as claimed in any one of claims 1 to 6,
**characterized in that** the internal toothset (16) of the outer gear (13) comprises five teeth at the most, whereas the external toothset of the inner gear (9) has, at most, fourth teeth (9D).

8. A heart prosthesis that can be implanted in the pericardial cavity of a patient, said prosthesis being capable of replacing the native left and right ventricles of said patient once these have been removed and comprising a body (30) in which artificial left and right ventricles (35) operated by at least one hydraulic actuator (37) are arranged,
**characterized in that** said hydraulic actuator is a displacement pump (1) like the one specified in any one of claims 1 to 7.

9. The heart prosthesis as claimed in claim 8, in which the artificial ventricles (35) comprise a cover plate (36) attached to said body (30),
**characterized in that** the displacement pump is connected to such a cover plate (36).

## Patentansprüche

1. Rotationsverdrängerpumpe, umfassend:
- ein Gehäuse (2), in dem eine Pumpenkammer (4) sowie ein radialer Fluideinlass (20) und -auslass (21) für die Pumpenkammer (4) angeordnet sind;
- ein äußeres Zahnrad (13) mit Innenverzahnung (16), das in der Pumpenkammer (4) angeordnet ist und die Form eines Käfigs aufweist, der mit zwei Endflanschen (14, 15) versehen ist, die durch Längsstäbe miteinander verbunden sind, die die Innenverzahnung bilden, wobei das äußere Zahnrad (13) mindestens teilweise demontierbar ist, um ein inneres Zahnrad einführen zu können;
- Drehmittel (11, 12), die die Drehung des äußeren Zahnrads (13) im Inneren der Pumpenkammer (4) um eine erste Achse (A13) ermöglichen und die mindestens ein Lager (11, 12) umfassen, das mittig in einem Endflansch (14, 15) des äußeren Zahnrads (13) angeordnet ist;
- das innere Zahnrad (9), das im Inneren des äußeren Zahnrads (13) angeordnet ist und mit einer Außenverzahnung (9D) versehen ist, die mit der Innenverzahnung (16) des äußeren Zahnrads (13) ineinandergreift, wobei sich das innere Zahnrad (9) um eine zweite Achse (A8) dreht, die parallel zu und beabstandet von der ersten Achse (A13) ist, und wobei die Außenverzahnung (9D) eine Anzahl an Zähnen aufweist, die um eine Einheit kleiner ist als die Anzahl der Zähne der Innenverzahnung des äußeren Zahnrads (13);
- einen Motor (3), um das innere Zahnrad (9) in eine Drehbewegung um die zweite Achse (A8) zu versetzen; und
- wobei die Pumpenkammer (4) eine abnehmbare Wand (10) umfasst, die geeignet ist, an dem Gehäuse (2) befestigt zu werden, um die Pumpenkammer (4) zu verschließen, und wobei ein erstes Lager (12) der Drehmittel in der abnehmbaren Wand (10) angeordnet ist;
**dadurch gekennzeichnet, dass**
- ein zweites Lager (11) der Drehmittel in einer feststehenden, einen integralen Bestandteil des Gehäuses bildenden Wand (5) der Pumpenkammer (4) angeordnet ist;
- das Gehäuse (2) eine Aufnahme (2M) für den Motor (3) umfasst und die feststehende Wand (5) die Aufnahme (2M) von der Pumpenkammer (4) trennt;
- die Welle (8) des Motors (3), der das innere Zahnrad (9) in eine Drehbewegung versetzt, die feststehende Wand (5) im Inneren des zweiten Lagers (11) durchquert; und
- die Welle (8) des Motors (3) hohl ist und mit radialem Spiel von einer Strebe (22) durchquert wird, die dazu dient, die abnehmbare Wand (10) am Gehäuse (2) zu befestigen.

2. Verdrängerpumpe nach Anspruch 1,
**dadurch gekennzeichnet, dass** mindestens einer der Endflansche (14, 15) des äußeren Zahnrads (13) demontierbar ist.

3. Verdrängerpumpe nach einem der Ansprüche 1 oder 2,
**dadurch gekennzeichnet, dass** Lager (23, 24) für das innere Zahnrad (9) an der Strebe (22) angeordnet sind.

4. Verdrängerpumpe nach Anspruch 3, die dazu bestimmt ist, in eine hydraulische Flüssigkeit eingetaucht zu werden,
**dadurch gekennzeichnet, dass** die Strebe (22) hohl ist, um die Flüssigkeit eindringen zu lassen und dass die Seitenwand der Strebe (22) mit Öffnungen (26) versehen ist.

5. Verdrängerpumpe nach einem der Ansprüche 1 bis 4,
**dadurch gekennzeichnet, dass** sich der radiale Fluideintritt (20) von einem Flansch des äußeren Zahnrads (13) zum anderen erstreckt.

6. Verdrängerpumpe nach einem der Ansprüche 1 bis 5,
**dadurch gekennzeichnet, dass** sich der radiale Fluidaustritt (21) von einem Flansch des äußeren Zahnrads (13) zum anderen erstreckt.

7. Verdrängerpumpe nach einem der Ansprüche 1 bis 6,
**dadurch gekennzeichnet, dass** die Innenverzahnung (16) des äußeren Zahnrads (13) höchstens fünf Zähne umfasst, während die Außenverzahnung des inneren Zahnrads (9) höchstens vier Zähne (9D) umfasst.

8. Herzprothese, welche in die Perikardhöhle eines Patienten implantierbar ist, wobei die Prothese geeignet ist, die natürliche linke und rechte Herzkammer des Patienten nach Ablation dieser zu ersetzen, und wobei sie einen starren Körper (30) umfasst, in dem eine künstliche linke und rechte Herzkammer (35) gebildet sind, die durch mindestens eine hydraulische Betätigungsvorrichtung (37) betätigt werden,
**dadurch gekennzeichnet, dass** die hydraulische Betätigungsvorrichtung eine Verdrängerpumpe (1) ist, wie sie in einem der Ansprüche 1 bis 7 spezifiziert wird.

9. Herzprothese nach Anspruch 8, wobei die künstlichen Herzkammern (35) einen Verschlussdeckel (36) umfassen, der an den Körper (30) angesetzt ist,
**dadurch gekennzeichnet, dass** die Verdrängerpumpe mit einem solchen Verschlussdeckel (36) verbunden ist.
